Europäisches Patentamt

European Patent Office

Office européen des brevets

(11) Publication number:

**0 312 358**
**A2**

(12) **EUROPEAN PATENT APPLICATION**

(21) Application number: **88309604.2**

(22) Date of filing: **13.10.88**

(51) Int. Cl.⁴: **C 07 K 3/08**
**C 12 P 21/02**

(30) Priority: **14.10.87 US 108433**

(43) Date of publication of application:
**19.04.89 Bulletin 89/16**

(84) Designated Contracting States:
**AT BE CH DE ES FR GB GR IT LI LU NL SE**

(71) Applicant: **PITMAN-MOORE, INC.**
**P.O. Box 207 1401 South Third Street**
**Terre Haute Indiana 47808 (US)**

(72) Inventor: **Seely, James E.**
**R.R. 25, 7389 Troy Court**
**Terre Haute Indiana 47802 (US)**

**Yang, Ren-der**
**4691 Springfield Place**
**Terre Haute Indiana 47808 (US)**

(74) Representative: **Holmes, Michael John et al**
**Frank B. Dehn & Co. Imperial House 15-19 Kingsway**
**London WC2B 6UZ (GB)**

(54) Method for promoting intramolecular disulfide bond formation in recombinant proteins contained in a denaturant solution.

㋙ A method is provided for promoting intramolecular disulfide bond formation in reduced recombinant proteins contained in a denaturant solution. Recombinant proteins which have been solubilized using a denaturant are reacted with an amount of the oxidizing agent cystine sufficient to oxidize free sulfhydryl groups and form intramolecular disulfide bonds.

EP 0 312 358 A2

Bundesdruckerei Berlin

Description

## METHOD FOR PROMOTING INTRAMOLECULAR DISULFIDE BOND FORMATION IN RECOMBINANT PROTEINS CONTAINED IN A DENATURANT SOLUTION

This invention relates generally to methods for promoting disulfide bond formation and particularly to a method for promoting intramolecular disulfide bond formation in reduced recombinant proteins contained in a denaturant solution.

## BACKGROUND OF THE INVENTION

Methods for producing recombinant proteins are well known in the art; DNA coding for the desired protein is inserted into a host microorganism which is grown under conditions which express the recombinant protein. These recombinant proteins are, however, not typically produced in an easily recoverable and bioactive form. The recombinant proteins are often produced in "inclusion bodies" which contain the protein in bio-inactive oligomers and aggregates. These inclusion bodies must be processed to produce a bioactive monomeric protein.

Methods for processing the "inclusion bodies" to recover the protein in bioactive form have varied. Generally, the insoluble inclusion bodies are recovered from the microorganism cell and dissolved in a strong denaturant or detergent. The resulting solution contains the desired protein in a denatured and reduced form. To obtain a bioactive protein, the tertiary configuration characteristic of the native molecule must be restored. This generally involves refolding the denatured protein and oxidizing the previously reduced sulfhydryl groups to form disulfide bonds which maintain the tertiary configuration, at the bioactivity characteristics of the recombinant protein.

Current methods for obtaining the bioactive tertiary configuration typically include removing the denaturant or detergent to allow the protein to refold into its bioactive conformation. This process generally includes a step in which reduced sulfhydryl groups are oxidized to form disulfide bonds which help to maintain the tertiary configuration of the molecule.

Renaturation of the protein and reforming of the correct disulfide bonds is a difficult and unpredictable process. When the denaturant is removed and the disulfide bonds are formed, tertiary configurations which are not consistent with the bioactivity of the native or recombinant molecule can be formed. Intermolecular disulfide bonds which produce oligomers and aggregates may form and destroy the bioactivity of the interlinked molecule. Also, incorrect intramolecular disulfide bonds which cause the molecule to assume a bio-inactive tertiary configuration may be formed.

Prior art methods for recovering the recombinant proteins and oxidizing the sulfhydryl groups to form disulfide bonds in recombinant molecules have varied. U.S. Patent Nos. 4,511,503 and 4,518,526 to Olson et al and U.S. Patent Nos. 4,511,502 and 4,620,948 to Builder et al disclose methods wherein inclusion bodies are solubilized in a strong denaturant and a reducing agent, contaminants are removed from the solubilized protein solution, the strong denaturant is replaced with a weak denaturant, and the protein is allowed to refold assisted by oxidation of the sulfhydryl groups to disulfide bonds using molecular oxygen and a catalyst, typically metal cations cr sodium tetrathionate. These methods for oxidizing sulfhydryl groups to form correct disulfide bonds, however, have the disadvantage of being very slow since the molecular oxygen is obtained from the atmosphere, typically requiring the solutions to sit exposed to the atmosphere for 3 to 8 days. Also, these methods generally form the disulfide bonds only after the strong denaturant has been diluted, removed, or replaced by a weaker denaturant to reduce its effect on the protein structure. Other disadvantages include the unpredictability of the reaction, chances of bacterial growth or other contamination of the solution while sitting exposed to the atmosphere, formation of oligmers and aggregates as the solution sets for long periods (generally longer than one week), and the like.

U.S. Patent No. 4,530,787 to Shaked et al discloses a method for oxidizing reduced cysteine-containing microbial proteins to produce synthetic proteins such as IL-2 in a controlled manner such that the synthetic proteins have the same disulfide bridging as their native counterparts. The oxidation employs o-iodosobenzoate as oxidizing agent.

U.S. Patent No. 4,569,790 to Koths et al discloses a process in which recombinant IFN-β and IL-2 proteins are selectively oxidized using o-iodosobenzoate to form the disulfide bonds corresponding to the native molecule.

U. S. Patent No. 4,572,798 to Koths et al discloses a process in which recombinant IFN-β and IL-2 proteins are selectively oxidized using $Cu^{+2}$ cations to form the disulfide bonds corresponding to the native molecule. The oxidation occurs in a solution containing a minimal amount of denaturant necessary to keep the protein in solution. Although the reaction using copper ions is reported to be very fast, about 1 hour, the process has a disadvantage of likely injecting copper ions into the solution thereby causing some undesirable interactions between copper ions and proteins. Additionally, the copper ions function as a catalyst which, due to the rapid formation of the disulfide bonds, produce incorrect intermolecular disulfide bcnds which cause bio-inactive oligmers and aggregates and produce incorrect intramolecular disulfide bonds which cause bio-inactive monomers that are useless for the desired purpose.

A method is, therefore, needed which can promote intramolecular disulfide bond formation in reduced recombinant proteins contained in denaturant solutions. This method should oxidize the sulfhydryl groups in a relatively short period of time, produce a high yield of bioactive, monomeric proteins, and reduce the presence of bio-inactive molecules caused by incorrect intramolecular disulfide bond formation and by intermolecular disulfide bond formation.

## SUMMARY OF THE INVENTION

It is, therefore, an object of the present invention to provide a method for promoting intramolecular disulfide bond formation in reduced recombinant proteins contained in a denaturant solution.

It is another object of the present invention to provide a method for promoting intramolecular disulfide bond formation in reduced recombinant proteins contained in a denaturant solution in a period of from about 6-48 hours.

It is another object of the present invention to provide a method for promoting intramolecular disulfide bond formation in reduced recombinant proteins contained in a denaturant solution using a method that produces a high yield of bioactive, monomeric recombinant proteins.

It is another object of the present invention to provide a method for promoting intramolecular disulfide bond formation in reduced recombinant proteins contained in a denaturant solution using a method that reduces the presence of bio-inactive recombinant proteins caused by incorrect intramolecular and intermolecular disulfide bond formation.

It is a further object of the present invention to provide a method for promoting intramolecular disulfide bond formation in recombinant somatotropins contained in a denaturant solution.

These and other objects are achieved by reacting recombinant proteins which have been solubilized using a denaturant to produce a solution of reduced, denatured protein with an amount of the oxidizing agent cystine sufficient to oxidize free recombinant protein sulfhydryl groups and form intramolecular disulfide bonds. The reaction results in a denaturant solution containing most of the recombinant protein in bioactive configuration that has the intramolecular disulfide bonds characteristic of the native protein and very little of the recombinant protein in a bio-inactive configuration caused by incorrect intramolecular and intermolecular disulfide bond formation.

In the preferred embodiments, recombinant sulfhydryl reduced proteins contained in a denaturant solution, generally sodium dodecyl sulfate (SDS), urea, or guanidine hydrochloride solutions, produced during the process of recovering bioactive proteins from inclusion bodies are reacted with an amount of cystine sufficient to oxidize recombinant protein sulfhydryl groups and form intramolecular disulfide bonds.

In the most preferred embodiment, a 0.1-2% SDS solution having a pH of from about 7-11 and containing from about 0.1-3.0 mg/ml of reduced recombinant somatotropin (rST) is reacted with from about 0.5-10 moles cystine per mole rST for about 12-36 hours to oxidize the rST sulfhydryl groups and form intramolecular disulfide bonds characteristic of native protein.

After the disulfide bonds have been formed, the protein solution is further processed to remove the denaturant and restore the bioactivity of the protein. Additional processing is often necessary to remove any remaining contaminants and produce a dosage composition suitable for administration to an animal.

Other objects, advantages, and novel features of the present invention will become apparent from the following detailed description of the invention.

## DESCRIPTION OF DRAWINGS

Figure 1 is a representation of SDS-PAGE gels showing the relative amount of reduced, oxidized, and aggregated recombinant porcine somatotropin (rpST) obtained using the method of the present invention.

Figures 2, 3, 4, and 5 are representations of Superose 12 elution profiles of the AG11A8 eluates showing the relative amount of monomeric recombinant porcine somatotropin (rpST) and high molecular weight contaminants obtained using the method of the present invention.

## DETAILED DESCRIPTION OF THE INVENTION

The term "recombinant protein" as used herein includes proteins having the amino acid sequence of the native protein and their analogs and muteins having substituted, deleted, replaced, or otherwise modified sequences. Also included are proteins which have one or more of the cystines in the amino acid sequence replaced with non-sulfur containing amino acids in an attempt to prevent incorrect disulfide bond formation.

The term "recombinant somatotropin" (rST) as used herein includes proteins having the amino acid sequence of native somatotropin, amino acid sequences substantially similar thereto, or an abbreviated sequence form thereof, and their analogs and muteins having substituted, deleted, replaced, or otherwise modified sequences. In particular, rST as used herein includes a protein of the same sequence as pST, but having amino acids deleted from it's amino terminal end. Examples of such proteins include but are not limited to delta-7 recombinant porcine somatotropin, delta-4 recombinant bovine somatotropin, and the like. Also included are rSTs which have cystines in the amino acid sequence replaced with non-sulfur containing amino acids in an attempt to prevent incorrect disulfide bond formation.

The term "denaturant" as used herein refers to those chaotropic compounds or materials which, in aqueous solution in suitable concentrations, are capable of reversibly changing the spatial configuration or conformation of proteins through alterations at the surface thereof, either through altering, for example, the state of hydration, the solvent environment, or the solvent-surface interaction. Examples of such denaturants include urea, guanidine hydro-

chloride, sodium thiocyanate, and detergents such as SDS.

The term "denaturant solution" refers to a solution which contains a "denaturant" as defined above. "Denaturant solution" is not defined to include high temperature, high acidity, and the like solutions which irreversibly denature a protein.

The terms "disulfide bond" and "disulfide bond formation" refer to intramolecular disulfide bonds and disulfide bond formation unless otherwise stated.

The term "intramolecular disulfide bond" and "intramolecular disulfide bond formation" refer to intramolecular disulfide bonds characteristic of the native protein unless otherwise stated.

According to the present invention, a method is provided for promoting disulfide bonds formation in reduced recombinant proteins contained in a denaturant solution. The method comprises reacting the recombinant protein with an amount of cystine sufficient to oxidize free protein sulfhydryl groups and form disulfide bonds. A high percentage, typically 50-70%, of the proteins reacted according to the present invention form disulfide bonds characteristic of the native protein. The resulting proteins are either bioactive once the disulfide bonds form or, assisted by the correct disulfide pairings, refold into a bioactive conformation when the denaturant is partially or completely removed from the solution.

The denaturant solutions of recombinant protein useful in the present invention are obtained by methods known in the art. Typically, protein inclusion bodies which have been produced by recombinant microorganisms are processed to remove contaminant proteins, lipids, and cell debris and the resulting relatively pure inclusion bodies containing mostly recombinant protein are solubilized in the denaturant to form a solution containing denatured, reduced recombinant protein.

Cystine, (i.e., o-diamidopropionic acid also commonly referred to as dicysteine) according to the present invention, is added to the denaturant solution before or after the inclusion bodies are solubilized in amounts sufficient to insure complete oxidation of the recombinant protein sulfhydryl groups, typically in amounts from about 0.5-10 moles of cystine per mole of recombinant protein, preferably from about 1-4 moles cystine per mole of recombinant protein. The amount may vary depending upon reaction conditions such as temperature, protein concentration, pH, concentration of denaturant or detergent, type of denaturant or detergent, time required for oxidation, and the like.

When adding the cystine after the inclusion bodies containing the recombinant protein have been solubilized, the denaturant concentration may be reduced prior to adding the cystine. Methods for removing or reducing the concentration of the denaturant are well known. For example, the denaturant concentration can be reduced or the denaturant can be removed by dialysis, cold precipitation, chromatography, and the like, although any well known method is acceptable.

The denaturant in the denaturant solution can be any denaturant suitable for recovering the recombinant protein from the inclusion bodies, typically sodium dodecyl sulfate (SDS), urea, or a guanidine salt such as guanidine hydrochloride.

The concentration of the denaturant varies depending upon the denaturant used: SDS concentrations range from about 0.05-5% by weight, preferably from about 0.1-2% by weight; urea concentrations range from about 5-9 M, preferably from about 6-8 M; and guanidine hydrochloride concentrations range from about 4-9 M, preferably from about 5-7 M.

The recombinant protein concentration in the denaturant solution is generally kept relatively low, from about 0.05-10 mg/ml, preferably 0.2-4 mg\ml, to reduce the likelihood of intermolecular disulfide bond formation and thus reduce the formation of oligmers and aggregates. The total protein concentration is generally from about 1-30 mg/ml, preferably from about 3-10 mg/ml.

The pH of the denaturant solution is maintained at from about 7-11, preferably from about 9-10, most preferably about 9.8. At lower pH, the oxidation reaction rate is considerably reduced. At higher pH, greater than 11, the protein may be susceptible to side-chain lesions, such as deamidation.

The reaction time needed for cystine to oxidize free sulfhydryl groups in the recombinant protein will depend upon the concentration of the denaturant and the reaction temperature; to a lesser extent the reaction time also depends upon the concentration of cystine, the concentration of recombinant protein, and the like. For reaction temperatures between about 0-30°C, at the concentration for the reagents given above, a reaction time of from about 6-48 hours, preferably from about 12-36 hours, is required to insure disulfide bond formation. This reaction time is in contrast to the 3-8 days required when protein solutions are allowed to sit exposed to air.

The recombinant protein useful in the present invention can be any protein which is produced by recombinant microorganisms and is solubilized by denaturants to produce denaturant solutions of the protein. These include somatotropins, prolactins, placental lactogens, and the like.

Most preferably, recombinant somatotropins (rST) are used in the present invention. The rST can be a ST from any species but are preferably bovine, porcine, avian, ovine, or human rST, most preferably porcine or bovine rST.

Methods for producing these recombinant proteins are well known in the art: For example, U.S. Patent Nos. 4,604,359 and 4,332,717 disclose methods for producing human recombinant somatotropin; U.S. Patent No. 4,431,739 discloses a method for producing recombinant somatotropin; E.P. Patent Application 0 104 920 discloses a method for producing recombinant porcine somatotropin; U.S. Patent No. 4,443,359 discloses a method for producing recombinant bovine somatotropin; E.P. Patent Application 0 103 395 discloses a method for producing recombinant bovine somatotropin; Schoner; Biotechnology, 3(2):151-54, discloses a method for producing recombinant somatotropin, and Buell, Nucleic Acid Res., 13, 1923-38 (1985) discloses a method for producing recombinant somatomedin C. Other methods for many similar proteins are known in the art.

In the preferred embodiment, SDS solutions of rST produced during the process of recovering bioactive somatotropins from inclusion bodies are reacted with an amount of cystine sufficient to oxidize sulfhydryl groups in the rST and form disulfide bonds. Most preferably, a 0.1-2% SDS solution having a pH of from about 7-11 and containing from about 3.0-10 mg/ml of reduced rST is reacted with from about 0.5-10 moles cystine per mole rST for about 12-36 hours to oxidize the rST sulfhydryl groups and form intermolecular disulfide bonds characteristic of native somatotropin.

After the disulfide bonds have been formed, the protein solution is further processed to remove any remaining contaminant proteins, solubilizing agents, oxidizing agents, reducing agents, protein oligmers and aggregates, and the like. Typically the solubilizing agent is removed by dialysis, chromatography, or other suitable means whereas the contaminating protein oligmers and aggregates are separated from the rST by chromatography. The oxidized rST is then further processed to produce a dosage composition suitable for administration to an animal to promote growth. These methods are well known in the art.

The invention having been generally described, the following examples are given as particular embodiments of the invention and to demonstrate the practice and advantages thereof. It is understood that the examples are given by way of illustration and are not intended to limit the specification or the claims to follow in any manner. In particular, inclusion bodies used in the experiments were prepared from transformed E. Coli strains which produce delta-7 porcine somatotropin. The inclusion bodies were isolated from E. Coli host strain HB101 transformed with a first plasmid (pL-mu-delta-7 pST) coding for delta-7 pST and a second plasmid (pCl 857) coding for the temperature sensitive lambda phage repression protein. Many other strains of microorganisms produce inclusion bodies containing many types of recombinant proteins which will function in the present invention. Similarly, methods for growing these microorganisms to produce inclusion bodies are well known in the art.

## Example 1

Washed inclusion bodies containing delta-7 porcine somatotropin produced from the E. Coli HB101 strain described previously were dissolved in 1% SDS/carbonate buffer (21 mM $Na_2CO_3$, 25 mM $NaHCO_3$, pH 9.8) at a ratio of 1 gram inclusion bodies per 40 ml of buffer solution in the presence of either 0.1 mM cupric sulfate, 0.1 mM L-cystine, 0.1 mM cystamine or no oxidizing agent (control). The samples were incubated in 15 ml Corex tubes with mild agitation at room temperature. After six hours of incubation, aliquots were taken from each sample, free sulfhydryl groups trapped with 30 mM iodoacetamide, and run on non-reducing SDS-PAGE gels to determine the relative amount of reduced vs oxidized and aggregated rpST. This method for determining the relative amount of reduced, oxidized, and aggregated recombinant protein on SDS-PAGE has been described previously for recombinant prochymosin (Schoemaker, et al. The EMBO Journal, 4(3) 775-780 (1985)). For rpST, the formation of the large disulfide loop, formed by oxidation of cystines 46 and 157 of delta-7 rpST, is accompanied by an increased mobility on SDS-PAGE relative to the reduced monomer (compare positions of bands 3 and 4 in Figure 1). Aggregated material, formed by intermolecular disulfide bond formation, migrates very slowly (band 1 in Figure 1). The remainder of the samples were incubated a total of 48 hours at room temperature. 0.75 ml aliquots were run over 1x10 cm AG11A8 columns in carbonate buffer to remove the SDS and the eluent run on size-exclusion-FPLC (Superose 12) to determine the amount of rpST monomer recovered. The results are shown in Figures 1 and 2.

Referring to Figure 1, both copper and cystine facilitate an increase in the oxidation rate of rpST from inclusions, as seen by the loss of reduced (slow mobility) and increase in the amount of oxidized (rapid mobility) rpST monomer. Cystamine, surprisingly, had no effect on the oxidation rate. Copper facilitated the accumulation of higher molecular weight aggregates as seen at the top of the gel. This indicates that although copper is a good oxidizing agent, it promotes a significant amount of intermolecular disulfide formation which produces bio-inactive oligmers and aggregates. Cystine, in contrast, did not facilitate the formation of higher molecular weight aggregates.

Referring to Figure 2, Figure 2A shows the Superose 12 elution profile for the control (no oxidizing agent), Figure 2B shows the elution profile for cupric sulfate (0.1 mM), Figure 2C shows the elution profile for L-cystine (0.1 mM), and Figure 2D shows the elution profile for cystamine (0.1 mM). The profiles are obtained by measuring the UV absorbance at 280 nanometers ($Abs_{280}$) as the protein is eluted from the column. Peaks from 6-12 minutes indicate contaminant proteins and the bio-inactive oligmers and aggregates of the recombinant protein. The 15 minute peak is the recombinant protein monomer which is to be recovered and purified. Comparing the 15 minute peaks for the desired protein, the profiles show that the peak for L-cystine is significantly larger, by about 30-50%, than the peak for the control, cupric sulfate, and cystamine. Therefore, although the rate of oxidation in the presence of copper is increased, much of the material is lost to covalent aggregation resulting in a decrease in the amount of monomer recovered.

## Example 2

Washed inclusion bodies containing delta-7 porcine somatotropin produced from the E. Coli HB101 strain described previously were dissolved in 1% SDS/carbonate buffer (21 mM $Na_2CO_3$, 25 mM $NaHCO_3$, pH 9.8) at a ratio of 1 gram inclusion bodies per 40 ml of buffer solution in the presence of 0.1 mM L-cystine. A control was prepared using the SDS buffer without the L-cystine. The samples were stirred 6 hours at room temperature (22°C) followed by a free SDS removal step in which the the free SDS was cold precipitated by incubating the solution at 5°C for 17 hours. The precipitate was removed by centrifugation at 15,000 x g for 15 min. At the end of the incubation period, protein bound SDS was removed by AG11A8 chromatography and samples analyzed for monomeric rpST on Superose 12 as described in Example 1. The results are shown in Figure 3.

Referring to Figure 3, Superose 12 elution profiles of the AG11A8 eluates for samples incubated in the absence (3A) and in the presence of (3B) 0.1 mM L-cystine show that the peak for L-cystine is 30-50% larger than the peak for the control. Cystine, therefore, increased the recovery of monomer in samples where the free SDS was removed by cold precipitation. Incubation of samples for an additional 48 hours after SDS removal failed to further increase recoveries of monomer over AG11A8 in both control and cystine samples.

## Example 3

Washed inclusion bodies containing delta-7 porcine somatotropin produced from the E. Coli HB101 strain described previously were dissolved in 1% SDS/carbonate buffer (21 mM $Na_2CO_3$, 25 mM $NaHCO_3$, pH 9.8) at a ratio of 1 gram inclusion bodies per 40 ml of buffer solution. The free SDS was cold precipitated as described in Example 2 by incubating at 5°C for 17 hours. The precipitate was removed by centrifugation at 15,000 x g for 15 min. Cystine was then added to produce a solution containing 0.1 mM L-cystine. At the end of a further 24 hour incubation period at room temperature, SDS was removed by AG11A8 chromatography and samples analyzed for monomeric rpST on Superose 12 as described in Example 1. The results are shown in Figure 4.

Referring to Figure 4, Superose 12 elution profiles of the AG11A8 eluates for samples incubated in the absence (4A) of and in the presence (4B) of 0.1 mM L-cystine show that the peak for L-cystine is 30-50% larger than the peak for the control. Cystine facilitates an increase in the amount of monomer recovered over AG11A8, although the amounts recovered here did not appear to differ significantly from experiments where cystine was present during cold incubation (Example 2).

## Example 4

Example 3 was repeated except that 0.025 mM cystine was used. The results are shown in Figure 5.

Referring to Figure 5, Superose 12 elution profiles of the AG11A8 eluates for 0.025 mM L-cystine (5A) and no oxidizing agent (5B-control) show that the peak for L-cystine is significantly larger than the peak for the control. A cystine concentration of 0.025 mM greatly increases the recovery (by over 30%) of rpST under these conditions, although 0.025 mM cystine was slightly less effective than 0.1 mM cystine (5C).

## Example 5

2.5 kilograms of washed inclusion bodies containing delta-7 porcine somatotropin produced from the E. Coli HB101 strain described previously were dissolved in 140 liters of 0.5% SDS/carbonate buffer (pH 9.8). The batch was split into two equal parts with part A allowed to air oxidize by slow agitation at 20°C and part B allowed to oxidize with the addition of 0.1 mM cystine. During the oxidation, samples were taken at intervals and run through small resin columns for SDS removal. The column eluate was analyzed for monomeric pST on a Superose 12 column. The results are shown in Table 1.

Referring to Table 1, the results indicate that the addition of cystine greatly enhances the rate of oxidation. Recovery of bioactive pST monomer reached its maximum in less than 24 hours after cystine addition.

## Example 6

6.75 kilograms of washed inclusion bodies containing delta-7 porcine somatotropin produced from the E. Coli HB101 strain described previously were dissolved in 270 liters of 0.5% SDS/carbonate buffer (pH 9.8) at 20°C for approximately 9 hours. The dissolute was chilled to ~4°C for about 15 hours to precipitate the free SDS. Following removal of the precipitated SDS by centrifugation using a Sharples centrifuge, the SDS-pST extract was allowed to air oxidize for about 21 hours before 0.1 mM cystine was added (to compare the effect of monomer recovery with the control). The monomer recovery was analyzed using a Superose 12 column following the SDS removal of the samples through an AG11A8 resin column. The results are shown in Table 1.

Referring to Table 1, the results indicate that cystine enhanced the rate of oxidation and, consequently, the monomer reaches its maximum in less than 6 hours following the addition of cystine.

Although incubation with cystine always gave an increased recovery of monomer under these conditions, it would likely not increase the net recovery compared to samples allowed to oxidize under various conditions for the optimal time period (greater than 1 week) in the absence of cystine. The advantage of using cystine is rather in its ability to accelerate the oxidation rate of rpST in the denaturant, particularly SDS. Incubation of extracts with cystine will decrease the oxidation time from greater than one week to less than two days. This increases through-put as well as prevents other problems associated with long-term oxidation (such as bacterial contamination). Also, cystine is very inexpensive and, at the concentrations required, adds insignificantly to production costs.

Obviously many modifications and variations of the present invention are possible in light of the above teachings. It is therefore to be understood that within the scope of the appended claims the invention may be practiced otherwise than as specifically described.

Table 1

Effect of Cystine on Oxidation and Monomer Yield

| Time After Beginning of Dissolution | GPC Monomer, ppm * | |
|---|---|---|
| | Part A (w/o Cystine) | Part B (w/Cystine) |
| 4 Hrs. | 311 | 392 |
| 1 Day | 415 | 515 |
| 2 Days | 430 | 488 |
| 3 Days | 454 | 425(?) |
| 4 Days | 445 | 492 |

*The sample was diluted 1:25 with carbonate buffer before running through SDS removal resin column.

Table 2

Effect of Cystine on Oxidation and Monomer Yield

| Days After Dissolution | GPC Monomer, ppm** | |
|---|---|---|
| | (w/o Cystine) | (w/Cystine) |
| 0.3 | 368 | -- |
| 1 | 364 | - |
| 2* | 383 | 423 |
| 5 | 403 | 410 |
| 6 | 415 | 428 |
| 7 | 395 | 423 |
| 8 | 404 | 398 |
| 12 | 410 | - |

*The first sample was analyzed 6 hours after cystine addition.
**The sample was diluted 1:2.5 with carbonate buffer before running through SDS removal resin column.

Claims

1. A method for promoting intramolecular disulfide bond formation in reduced recombinant proteins contained in a denaturant solution, which comprises:reacting the recombinant proteins with an amount of cystine sufficient to oxidize free recombinant protein sulfhydryl groups and form the intramolecular disulfide bonds.

2. The method of claim 1 wherein the denaturant solution is a 0.05-5% by weight SDS solution, a 5-9 urea solution, or a 4-9 M guanidine hydrocholoride solution.

3. The method of claim 1 or claim 2 wherein the denaturant solution is a 0.1-2% by weight SDS solution.

4. The method of any one of claims 1 to 3 wherein the recombinant protein concentration in the denaturant solution is from about 0.50-10 mg/ml.

5. The method of any one of claims 1 to 4 wherein the concentration of cystine in the denaturant solution is from about 0.5-10 moles of cystine per mole of recombinant protein.

6. The method of any one of claims 1 to 5 wherein the pH of the denaturant solution is maintained at from about 7-11.

7. The method of any one of claims 1 to 6 wherein the cystine is allowed to react with the recombinant protein for from about 6-48 hours.

8. The method of any one of claims 1 to 7 wherein the cystine is allowed to react with the recombinant protein for from about 12-36 hours.

9. The method of any one of claims 1 to 8 wherein the recombinant protein is a somatotropin, prolactin, or placental lactogen.

10. The method of claim 9 wherein the recombinant protein is an avian, ovine, or human recombinant somatotropin.

11. The method of claim 9 wherein the recombinant somatotropin (rST) is a porcine or bovine recombinant somatotropin.

12. The method of any one of claims 9 to 11 wherein the rST concentration in the denaturant solution is from about 1-3 mg/ml.